# EUROPEAN PATENT SPECIFICATION

(11) **EP 4 157 247 B1**
(45) Date of publication and mention of the grant of the patent: **30.07.2025**
(21) Application number: 21731565.4
(22) Date of filing: 25.05.2021
(51) Int. Cl.: A61K 31/145, A61K 45/06, A61P 29/00, A61P 31/12, A61P 31/14

(54) **CYSTEAMINE FOR USE IN ANTI-VIRAL THERAPY**
CYSTEAMINE ZUR VERWENDUNG IN DER ANTIVIRALEN THERAPIE
CYSTEAMINE POUR SON UTILISATION DANS UN TRAITEMENT ANTI-VIRAL

(30) Priority: 25.05.2020 GB 202007768; 07.08.2020 GB 202012298; 15.02.2021 GB 202102106
(43) Date of publication of application: 05.04.2023
(73) Proprietor: Novabiotics Limited, Aberdeen AB23 8EW (GB)
(72) Inventor: O'NEIL, Deborah, Aberdeen Aberdeenshire AB23 8EW (GB); FRASER-PITT, Douglas, Aberdeen Aberdeenshire AB23 8EW (GB)
(74) Representative: HGF
(86) International application number: PCT/GB2021/051269
(87) International publication number: WO 2021/240145

(56) References cited:
- WO-A1-2007/062272
- WO-A2-2010/138419
- US-A- 5 554 655
- US-A- 5 646 189
- US-A1- 2011 053 894
- PAUL MCINTOSH: "NovaBiotics announces fast-track repurposing of its experimental drug Nylexa for COVID-19 trials and plans for earliest possible compassionate use", 13 April 2020 (2020-04-13), XP055832011, Retrieved from the Internet <URL:https://novabiotics.co.uk/novabiotics-announces-fast-track-repurposing-of-its-experimental-drug-nylexa-for-covid-19-trials-and-plans-for-earliest-possible-compassionate-use/> [retrieved on 20210813]
- KANDEEL MAHMOUD ET AL: "Virtual screening and repurposing of FDA approved drugs against COVID-19 main protease", LIFE SCIENCE, PERGAMON PRESS, OXFORD, GB, vol. 251, 3 April 2020 (2020-04-03), XP086144978, ISSN: 0024-3205, [retrieved on 20200403], DOI: 10.1016/J.LFS.2020.117627
- PULMONOLOGY PEDIATRIC ET AL: "Poster Session Abstracts", PEDIATRIC PULMONOLOGY., vol. 53, no. S2, 1 September 2018 (2018-09-01), US, pages S148 - S456, XP055832008, ISSN: 8755-6863, Retrieved from the Internet <URL:https://eprints.whiterose.ac.uk/137728/3/NACF%20Abstract%20July%202018.pdf> DOI: 10.1002/ppul.24152
- ANONYMOUS: "NovaBiotics Announces New Data on Cysteamine (Nylexa(TM)) as an Antimicrobial Resistance Breaker in Multi Drug Resistant Bacteria", BUSINESS WIRE, 18 June 2016 (2016-06-18), pages 1 - 3, XP055400270, Retrieved from the Internet <URL:http://www.businesswire.com/news/home/20160618005003/en/NovaBiotics-Announces-New-Data-Cysteamine-Nylexa(TM)-Antimicrobial> [retrieved on 20170822]
- KHANNA KRITIKA ET AL: "Binding of SARS-CoV-2 spike protein to ACE2 is disabled by thiol-based drugs; evidence from in vitro SARS-CoV-2 infection studies", BIORXIV, 8 December 2020 (2020-12-08), pages 1 - 22, XP055799010, Retrieved from the Internet <URL:https://www.biorxiv.org/content/10.1101/2020.12.08.415505v1.full.pdf> [retrieved on 20210427], DOI: 10.1101/2020.12.08.415505

## Description

The present invention relates to the use of compounds, and compositions comprising such compounds, for treating viral infection. More specifically, the compounds for use in the present invention are cysteamine or cystamine, or a pharmaceutically acceptable salt thereof. As an example, the compounds may be used for treating infection with a coronavirus, e.g. acute respiratory syndrome coronavirus 2 (SARS-CoV-2) infection, known as COVID-19.

### BACKGROUND

Viral infection in the human and animal populations is not a new problem. However, with the emergence of a more global society, the ability for particularly virulent infections to spread to the point of epidemic or pandemic proportions is becoming more common. In 2002-2003 the world experienced infection with Severe Respiratory Syndrome (SARS) caused by SARS-associated coronavirus (SARS-CoV), in 2012 it was the turn for infection with the Middle East Respiratory Syndrome (MERS) caused by Middle East Respiratory Syndrome Coronavirus (MERS-CoV). In 2019-2020, we have seen the emergence of an even more transmissible viral infection in the form of Coronavirus disease 2019 (COVID-19), caused by SARS-Associated Coronavirus 2 (SARS-CoV-2).

As the identification of sufficiently effective vaccines and therapeutic treatments of coronavirus infections, such as SARS-CoV-2, has thus far eluded science, the principle strategy to halt such pandemics has been to "lock-down" society, thereby using social distancing to prevent transmission of the virus. Such strategies are clearly incredibly detrimental to world economies, our way of life and the population's mental health in general. There is therefore a need for new therapeutics that can target the treatment, including prophylactically, of viral infections in general, and currently those relating to coronavirus infection. This is particularly an urgent requirement for emerging viral infection, such as COVID-19. Approximately 5% of patients infected with COVID-19 require admission to Intensive Therapy Units (ITUs) and require mechanical ventilatory support because of respiratory failure, the mortality rate for these patients is 50-60%, in addition many patients will require prolonged stay in ITU (>14 days) further increasing pressure on ITU resources. The pathological processes resulting in respiratory failure are not entirely clear but are likely to include direct damage to bronchial epithelial/alveolar cells by COVID-19, superadded bacterial infection and associated inflammatory reaction. There is currently no approved therapeutic composition in Europe or the US for treating coronavirus infection.

Clinical interventions designed to directly address viral infection essentially fall into three categories; (1) antiviral (2) viricidal therapeutics or (3) vaccines. Viricidal therapeutics deactivate virions and so are aimed at destroying the viral infection. Antiviral therapeutics are aimed at interrupting viral replication (for example, Acyclovir). A vaccine is designed to activate the acquired immune responses of the body to the viral invasion by stimulating B lymphocytes, thereby invoking an antibody response (e.g. IgA, IgM, or IgG). Each of these strategies can present difficulties. Designing an effective vaccine is a process fraught with technical hurdles and often takes a long time to arrive at an effective preparation. Viricidals often have off-target effects that can result in damage to the host and so are seldom developed for therapeutic purposes. Perhaps the most successful antiviral drug strategies are to administer non-functional "analogues" of the nucleotides that are used by the virus during replication, which block viral polymerase and so replication itself. Acyclovir, ribavirin and azidothymidine (AZT) are examples of these drugs and have been demonstrated to be effective at treating herpes simplex virus, hepatitis C and HIV/AIDS. Unfortunately, coronavirus can resist such strategies as it "proofreads" and edits out non-authentic nucleotides during replication. A further problem associated with both antiviral and viricidal therapeutics is that for optimum effect they often have to be administered directly to the site of infection. For example, in the case of COVID-19 this would normally be the lungs. Administering therapeutics to the lung is difficult and often painful.

Cystamine (2,2'-Dithiobis(ethylamine)) has been suggested to have radioprotective properties and to have potential for treating Huntington's disease. Cysteamine (2-Aminoethanethiol) is an aminothiol that possesses a number of characteristics that are currently used for therapeutic benefit. Cysteamine has been licensed for over 30 years for the treatment of the inherited metabolic disorder cystinosis, and in development for more than 16 years for the bacterial respiratory infections associated with of cystic fibrosis. Cysteamine has been shown to have powerful antibacterial (including antivirulence and antibiofilm), antibiotic-potentiating, mucolytic and anti-inflammatory properties.

Some limited work has been carried out on the potential use of cysteamine for treating HIV/AIDS and specific influenzas. In these studies, it was concluded that the cysteamine has antiviral effects on HIV by its cleavage of the envelope glycoprotein GP120 that is specific to HIV. Similar effects were found in connection with the treatment of specified influenza viruses. Cysteamine is therefore considered to act as a viricidal agent that targets envelope glycoproteins that are specific for these two classes of virus (ie HIV, H1N1 and H7N9). As the envelope glycoprotein populations are specific to each class of virus, it is not possible to have any reasonable level of expectation that cysteamine may be able to act on any other given virus.

Kandeel Mahmoud et al, Life Science, vol. 251,3 April 2020 (2020-04-03), 117627 describes docking of cysteamine onto COVID-19 viral protease.

To-date, there has been no empirical evidence that demonstrates that cysteamine, has any antiviral effect on a coronavirus infection.

### BRIEF SUMMARY OF THE DISCLOSURE

The applicants have been investigating the therapeutic utility of a number of compounds in respiratory and bacterial and fungal infectious disease for over 16 years and have a unique insight into the therapeutic potential that such compounds have to offer. Much of the work has been involved in elucidating the mechanisms of action of the compounds when directed to bacterial or fungal infections; mechanisms that have no relevance to antiviral activity. As discussed above, cysteamine has been proposed as having antiviral activity, but the results to-date, and explanations for mode of action, only point to the therapeutic effect being restricted to specific viruses. It has therefore been surprisingly found by the applicants that cysteamine or cystamine, are able to treat a coronavirus infection and indeed viral infection in general.

Accordingly, in a first aspect of the present invention, there is provided a compound comprising cysteamine or cystamine, or a pharmaceutically acceptable salt thereof, for use in the treatment of viral infection, by inhibiting the activity of glycine decarboxylase.

The viral infection can be caused by any virus, excepting HIV, H1N1, or H7N9. The virus may therefore be influenza virus, except H1N1, or H7N9. Therefore, the virus can be an influenza C virus. The virus can be an influenza virus with a haemagglutinin content that does not include H1 and is not H7N9, unless optionally the neuraminidase is not N1. Consequently, the virus can have a haemagglutinin content of any of H2-17, or H1 when the virus does not including N1, and is not H7N9. Consequently, the virus can have a neuramaninidase content of any of N2-9, or N1 when the virus does not include H1 and is not H7N9. The virus can be an influenza virus with a haemagglutinin content that does not include H7 and is not H1N1, unless optionally the neuraminidase is not N9. Consequently, the virus can have a haemagglutinin content of any of 1-6 or 8-17, or H7 when the virus does not including N9, and is not H1N1. Consequently, the virus can have a neuramaninidase content of any of N1-8, or N9 when the virus does not include H7 and is not H1N1. The viral infection can be a Ribovirus (but not H1N1, H7N9). The viral infection can be any of ebola, coronavirus, hepatitis C, hepatitis E , West Nile Fever, Norovirus, Rotavirus, Polio, rabies, measles, rhinovurus. The virus may be a coronavirus. For example, any virus of the family Coronaviridae. Such viruses are characterised as enveloped viruses with a positive-sense single-stranded RNA genome and a nucleocapsid of helical symmetry. The genome size of coronaviruses may be rather large, ranging from about 26 to 32 kilobases. The virus can be SARS-CoV, MERS-CoV , SARS-CoV-2, HCoV-299E, HCoV-OC43, HCoV-NL63, CoV-HKU1, Human Coronavirus 229E (ATCC No. VR/740) or any combination thereof. The virus is preferably SARS-CoV-2. SARS-CoV-2 is a betacoronavirus with 79% genetic homology with SARS-CoV, and 98% homology to the bat coronavirus RaTG13 (Zhou et al. A pneumonia outbreak associated with a new coronavirus of probable bat origin, Nature, 2020;579 (7798):270-3). It is spread in respiratory droplets and infects nasal, bronchial and alveolar epithelial cells by binding of the viral spike protein to its cellular receptor, ACE2 (Walls et al. Structure, Function, and Antigenicity of the SARS-CoV-2 Spike Glycoprotein. Cell. 2020;181(2):281-92 e6). Owing to the error prone nature of the viral replication process, RNA viruses such as SARS-CoV-2 accumulate mutations resulting in some sequence diversity. Nevertheless, different strains of SARS-CoV-2 can be recognised by sequencing and phylogenetic sequence trees. Exemplification of such phylogenetic tree analysis with rapid sequencing of isolates is reported for example by Meredith et al. Rapid Implementation of SARS-CoV- 2 sequencing to investigate cases of health-care associated COVID-19: a prospective genomic surveillance study in The Lancet , published on-line 14th July 2020. Sequences of amplified SARS virus genome can be compared with the NCBI Reference sequence NC_045512.2 or equivalent GenBank reference MN908947.3 for SARS-CoV-2 corresponding to the SARS-CoV-2 isolate Wuhan- Hu-1 complete genome (Wu et al. Nature 579, 265-269). SARS2- CoV-2 variant strains can thus be recognised and can be expected to have high homology to the reference genome, e.g. at least 90%, at least 95%, at least 98% or at least 99%. Such sequencing surveillance can equally enable any new SARS virus infecting humans to be identified. The use of compounds of the present invention may therefore be used for treating humans infected with any SARS-CoV virus, especially any SARS-CoV-2 viral strain (which can include variants thereof).

The applicants have surprisingly discovered that the antiviral mode of action for the compounds of the present invention (eg when addressed to a coronavirus infection) is different to the general known antiviral (eg anti-HIV/AIDS) mechanisms of action of some of those compounds. The applicant has determined that it is the action of the compound to inhibit the activity of glycine decarboxylase in the host that results in the anti-coronavirus effect. Based on their further investigations, this has enabled the applicants to identify a number of further inventive concepts associated with the use of the compounds of the present invention for treating viral infections.

Because glycine decarboxylase is ubiquitous in viruses, the realisation that, for example, coronavirus infection can be treated with compounds of the present invention in this manner supports the understanding that all viruses are treatable in this manner.

Consequently, in one aspect of the present invention, there is a provided a compound comprising cysteamine or cystamine, or a pharmaceutically acceptable salt thereof, for use in treating viral infection by inhibiting the activity of glycine decarboxylase. The viral infection may be that defined in connection with other aspects of the present invention.

Those subjects with an elevated activity level of glycine decarboxylase are as a result of the above understanding particularly susceptible to treatment with cysteamine for viral infection. Consequently, in a further aspect of the present invention, there is provided a compound comprising cysteamine or cystamine, or a pharmaceutically acceptable salt thereof, for use in treating viral infection in a subject, wherein subject has a glycine decarboxylase activity that is greater than normal. Determining such a subject may be achieved by a quantitative analysis of any biomarker for glycine decarboxylase activity, for example that found in the urine or blood. A lower level for that biomarker for the subject than normal would be indicative of an individual that would respond well to treatment for viral infection by the compounds of the present invention. Determining when the level is lower than normal may be possible by comparing the level for the biomarker with that in a sample provided by the subject to be treated prior to infection. If such samples are not available, it is well within the skill of a clinician to determine if the level for any individual biomarker is lower than normal. As an example, the biomarker can be glycine. The viral infection may be that defined in connection with other aspects of the present invention.

Consequently, in a further aspect of the present invention, there is provided a compound comprising cysteamine or cystamine, or a pharmaceutically acceptable salt thereof, for use in treating viral infection in a subject, wherein subject has a plasma concentration of glycine that is lower than or equal to 300, 290, 280, 250, 200, 150, 125 µmol/L . A preferred range is lower than or equal to 300 µmol/L. A further preferred range is lower than or equal to 125 µmol/L. The viral infection may be that defined in connection with other aspects of the present invention.

Therefore, before administering any of the compounds described, a subject may be tested for glycine levels in any bodily fluid, for example, blood (e.g. plasma) or urine. Glycine levels vary according to age and health. The skilled person, for example, a physician, would be aware of the appropriate levels of glycine for a subject. Example ranges are given below for guidance:

| **Age** | **Glycine levels in plasma by age in µmol/L** |
|---|---|
| Infants 1-3 months | 164 +/- 29 |
| Infants 3 to 6 months | 175-296 |
| Infants 9 months -2 years | 56-308 |
| Children 3-18 years | 117-302 |
| Adults over 18 years | 120-554 |
| | |

| **Condition** | **Glycine levels in plasma according to condition (with the mean for healthy individuals around 220 µmol/L (mean men at 211 µmol/L and mean women at 230 µmol/L)** |
|---|---|
| Type 2 diabetes | Mean women with T2DM: 184 |
| | Mean men with T2DM: 187 |
| Obesity | Mean women with obesity: 203 |
| | Mean men with obesity: 186 |

| **Age** | **Glycine levels in urine by age in µmol/L /day** |
|---|---|
| Infants: 10 days to 7 weeks | 194-787 |
| Children: 3-12 years | 165-1420 |
| Adults | 785-3918 |

In a non-claimed aspect, there is therefore also disclosed a method of: a) determining the level of glycine in a sample obtained from a subject; and b) comparing the level of glycine to a control range, wherein if the level of glycine is lower than the control or at a low level compared to the control the subject is treated with cysteamine or any of the compounds described below. By low level is meant, for example, the glycine may be in the lower 10% of the control range.

Alternatively, the glycine may be in the lower 15% of the control range.

Alternatively, the glycine may be in the lower 20% of the control range.

Alternatively, the glycine may be in the lower 25% of the control range.

Alternatively, the glycine may be in the lower 30% of the control range.

Alternatively, the glycine may be in the lower 35% of the control range.

Alternatively, the glycine may be in the lower 40% of the control range.

Alternatively, the glycine may be in the lower 45% of the control range.

Alternatively, the glycine may be in the lower 50% of the control range.

The control range in plasma for healthy adults is 120-554 with average levels of between 200-300 µmol/L. Therefore, the glycine may be below 120 or from 120-200 µmol/L. Alternatively, the glycine may be below 120 or from 120-150 µmol/L.

Alternatively, the glycine may be below 200-300 µmol/L. For example, below 300, 290, 280, 270, 260, 250, 240, 230, 220, 210, 200, 190, 180, 170, 160, 150 µmol/L.

For obese or type II diabetic subjects, the glycine may be below 190, 180, 170, 160, 150, 140, 130 or 120 µmol/L.

Other biomarkers which would identify patients eligible for treatment include: glycine decarboxylase or ammonia (which is a by-product of the glycine cleavage pathway).

Therefore, also disclosed is a method of: a) determining the activity of glycine decarboxylase in a sample obtained from a subject; and b) comparing the activity to a control range, wherein if the activity is higher than the control, the subject is treated with cysteamine of any of the compounds described below. The skilled person would be aware of normal activity levels of glycine decarboxylase in a subject. Also disclosed is a method of: a) determining the level of ammonia in a sample obtained from a subject; and b) comparing the level to a control range, wherein if the level is higher than the control, the subject is treated with cysteamine or any of the compounds described below. The normal range for ammonia in the blood is 15 to 45 µ/dL (11 to 32 µmol/L).

Other markers for increased flux through the glycine cleavage pathway may also be tested. Therefore, there is also disclosed a general method of a) determining the activity or level of a biomarker which indicates increased flux through the glycine cleavage pathway; and administering a compound described below. For example, any of the compounds below, for use in a method of treating a viral infection, wherein the method comprises: a) determining the activity or level of a biomarker which indicates increased flux through the glycine cleavage pathway; and b) administering the compound.

As well as testing for glycine or ammonia levels, or glycine decarboxylase activity, a physician may instruct the tests to be carried out.

A number of conditions are associated with an elevated level of activity of glycine decarboxylase, for example metabolic syndrome, diabetes (particularly type II) and obesity. The elderly (eg over 75) also tend to have elevated levels of glycine decarboxylase, particularly when suffering from dementia. Consequently, the compositions of the present invention may have particular utility when directed for use in treating viral infection in a subject, wherein subject is a diabetic (particularly type II), obese, suffers from dementia or elderly (particularly with dementia), or a combination thereof. The viral infection may be that defined in connection with other aspects of the present invention.

The applicant has furthermore identified that compounds capable of inhibiting the reaction that is catalysed by glycine decarboxylase *in vivo* (ie glycine cleavage) are able to optimise the anti-viral activity of cysteamine, particularly those that achieve such inhibition through means that does not involve inhibit glycine decarboxylase activity. For example, a suitable compound is aminooxyacetic acid or bicarbonate. Preferably the compound is bicarbonate.

Consequently, in a further aspect of the present invention there is a provided a composition comprising a compound comprising cysteamine or cystamine, or a pharmaceutically acceptable salt thereof, and an inhibitor of glycine cleavage, eg bicarbonate. It is preferred that such in inhibitors carry out this activity via a mechanism other than the inhibition of glycine decarboxylase. As such a composition has therapeutic value, the present invention may relate to a pharmaceutical composition comprising a compound comprising cysteamine or cystamine, or a pharmaceutically acceptable salt thereof, and an inhibitor of glycine cleavage (eg bicarbonate) and one or more pharmaceutically acceptable excipient. In a further aspect of the present invention, there is provided a composition comprising a compound comprising cysteamine or cystamine, or a pharmaceutically acceptable salt thereof, and bicarbonate for use in therapy. The compound and inhibitor of glycine cleavage (eg bicarbonate) may be prepared for separate, sequential or simultaneous administration. That therapeutic use may be the treatment of viral infection. The viral infection may be that defined in connection with other aspects of the present invention.

The compound of the present invention is cysteamine, a prodrug thereof (ie cysteamine produced in vivo by a subject being treated), or a pharmaceutically acceptable salt thereof. The prodrug is cystamine.

The compound of any of the aspects of the present invention may be provided as a composition with antiviral therapeutic activity. Consequently, it maybe that the composition comprises the compound as the only compound with antiviral activity in the composition. The composition may consist of the compound, optionally with additional pharmaceutically acceptable excipients.

Opportunistic bacterial infection often follow infection from viruses, not least infections associated with coronavirus, which can, on top of the viral infection, exacerbate adverse immunological responses further; pushing the subject closer to very serious conditions such as cytokine shock syndrome. Only treating viral infection will leave the bacterial infection to flourish, and vice versa if one were to treat the bacterial infection alone. Combinations therapies can be unpredictable. Consequently, a surprising advantage of the work carried out by the applicant is that one can treat a bacterial and viral infection with a single therapeutic that is effective for both, eg one of cysteamine or cystamine, or a pharmaceutically acceptable salt thereof. Immuno-suppressive activity of compounds of the present invention aids further in the reduction in progression of the deleterious effects of both such infections. Consequently, the compounds or compositions of the present invention show surprising utility for treating a viral infection (such as coronavirus) and bacterial infection simultaneously. Treating a subject with only viral (eg coronavirus) infection will also have the benefit of providing prophylactic treatment of the bacterial-co-infection. The subjects to be treated in the present invention may therefore be those with a viral and bacterial infection, or those with a viral infection and that may be vulnerable to a bacterial infection (for example, due to weekend state from a severe viral infection, or their presence in a hospital). The viral infection may be that defined in connection with other aspects of the present invention.

Because conventional antiviral therapeutics work in a different way to that of the present invention, co-administration of compounds or compositions of the present invention and a further antiviral therapeutic can provide an improved level of efficacy compared to the use of either therapeutic alone, for example, a synergistic level of improvement. This advantage can be found when combined with any conventional anti-viral therapeutic, eg ones that does not act via inhibition of glycine decarboxylase. As an example an additional antiviral agent may be Idoxuridine, Trifluridine, Brivudine, Vidarabinea, Entecavir, Telbivudine, Foscarnet, Zidovudine, Didanosine, Zalcitabinea, Stavudine, Lamivudine, Abacavir, Emtricitabine, Nevirapine, Delavirdinea, Efavirenz, Etravirine, Rilpivirine, Saquinavir, Ritonavir, Indinavir, Nelfinavir, Amprenavira, Lopinavir-ritonavir, Atazanavir, Fosamprenavir, Tipranavir, Darunavir, Telaprevira, Boceprevira, Simeprevir, Asunaprevirb, Paritaprevirb, Grazoprevirb, Raltegravir, Elvitegravir, Dolutegravir, RSV-IGIVa, Palivizumab, Docosanol, Enfuvirtide, Maraviroc, VZIGa, VariZIG, Acyclovir, Ganciclovir, , Famciclovir, Valacyclovir, Penciclovir, Valganciclovir, Cidofovir, Tenofovir disoproxil fumarate, Adefovir dipivoxil, Amantadinea, Ribavirin, Rimantadine, Zanamivir, Oseltamivir, Laninamivir octanoate, Peramivir, Favipiravir, Pegylated interferon alfa 2b, Interferon alfacon 1a, Fomivirsena, Podofilox, Imiquimod, Sinecatechins, Remdesivir, Flavipiravir, or any combination thereof. A preference may be for Remdesivir, Flavipiravir, Lopinavir-ritonavir or any combination thereof.

Consequently, compositions for use in any aspect of the present invention may include one or more further antiviral therapeutic. Alternatively, the compounds or compositions of the present invention may be administered separately, but as part of the same regimen, for treating viral infection (optionally coronavirus). The compositions or compounds of the present invention may therefore be provided for separate, simultaneous or sequential use with one or more further antiviral therapeutic for use in treating viral infection (optionally coronavirus).

The compositions of the present invention may be prepared for administration intravenously, orally, rectally, parentally, topically. Optionally, the compositions are prepared for administration to the site of infection, for example the lungs. In a further aspect of the present invention, there is provided a method of treating viral infection in a subject, the method comprising the step of administering any of the preceding compounds or compositions in a therapeutically effective amount to the subject. The viral infection maybe a coronavirus infection, as defined above. The method may be prophylactic treatment.

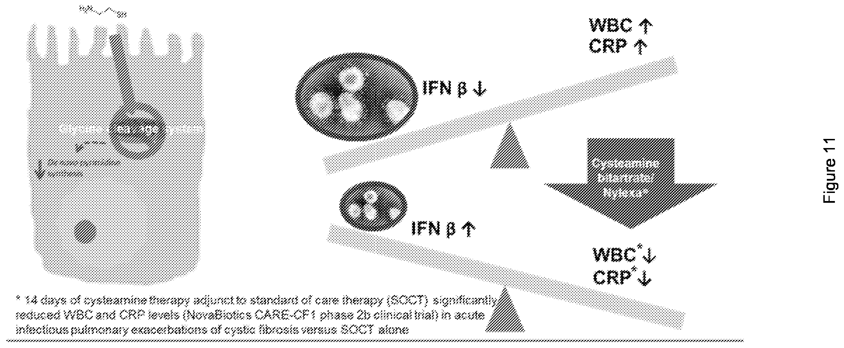

The inventors have also seen beneficial reduction in the damaging IL-6 induced inflammatory response as a result of cysteamine treatment (see the discussion of Figure 11 in Example 2 below where cysteamine's effect downstream from the glycine cleavage system is explained). Therefore, there is also provided cysteamine for use in a method of treating hyperinflammation. The hyperinflammation may be the result of viral infection or the result of bacterial or fungal infection. For example, the hyperinflammation may be the result of pneumonia caused by a viral or bacterial infection. The hyperinflammation may be in Acute respiratory distress syndrome (ARDS). The hyperinflammation may be in sepsis or toxic shock syndrome. The hyperinflammation may be as a result of other causes, i.e. not infection. For example, the hyperinflammation may be in an autoimmune disease, for example in a flare up of an autoimmune disease. An autoimmune flare-up is a period of worsening and intensification of symptoms. This can be diagnosed by using various biomarkers depending on the autoimmune condition. The autoimmune disease may be rheumatoid arthritis, systemic lupus erythematosus or inflammatory bowel disease (IBD). Hyperinflammation is systemic, acute inflammation and can be diagnosed by elevated levels of proinflammatory cytokines. Current treatments include IL-6 receptor antibodies and steroids. Patients have an increased incidence of shock, multiorgan failure and higher mortality. Hyperinflammation may also be described as hyperinflammatory disease or syndrome. Alternatively it may be referred to as systemic inflammatory response syndrome (SIRS).

The invention also relates to a pharmaceutically acceptable salt of the compound. Examples of pharmaceutically acceptable salts are discussed in Berge et al., 1977, "Pharmaceutically Acceptable Salts," J. Pharm. Sci., Vol. 66, pp. 1-19. If the compound is cationic, or has a functional group which may be cationic, then a salt may be formed with a suitable anion or a conjugate acid thereof. Examples of suitable inorganic anions include chloride, bromide, iodide, phosphate, dihydrogenphosphate, sulfate, bisulfate, hemisulfate, persulfate, sulfonate, and nitrate. Examples of suitable organic anions include acetate, adipate, alginate, aspartate, benzoate, benzenesulfonate, butyrate, camphorate, camphorsulfonate, cinnamate, citrate, crotonate, cyclopentanepropionate, detartrate, digluconate, disuccinate, dodecylsulfate, ethanesulfonate, fumarate, gluconate, glucoheptanoate, glycerophosphate, glycolate, heptanoate, hexanoate, 2-hydroxyethane-sulfonate, lactate, maleate, mandelate, methanesulfonate, 2-naphthalenesulfonate, nicotinate, oxalate, pamoate, pantothenate, pectinate, picrate, pivalate, propionate, salicylate, succinate, tartrate, thiocyanate, tosylate, tropate, trichloroacetate, undecanoate, and undecylenate.

If the compound is anionic, or has a functional group which may be anionic, then a salt may be formed with a suitable cation. Examples of suitable inorganic cations include alkali metal ions, such as Na⁺ and K⁺, and the alkaline earth metal cations, such as Ca²⁺ and Mg²⁺. Examples of suitable organic cations include ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, triethylamine, as well as amino acids, such as lysine and arginine.

Generally, it is preferred that the pharmaceutically acceptable salt of the compound is a chloride salt or a bitartrate salt.

The compound may be cysteamine bitartrate.

cysteamine or cystamine, according to any aspect of the present invention will inhibit the activity of glycine decarboxylase activity. How to determine such inhibition is described above.

Where the context permits, all optional features of the first aspect of the present invention apply equally to the further aspects of the present invention.

Throughout the specification, unless the context demands otherwise, the terms 'comprise' or 'include', or variations such as 'comprises' or 'comprising', 'includes' or 'including' will be understood to imply the method or kit includes a stated integer or group of integers, but not the exclusion of any other integer or group of integers.

Aspects of the present invention will now be illustrated by way of example only and with reference to the following experimentation and to the figures.

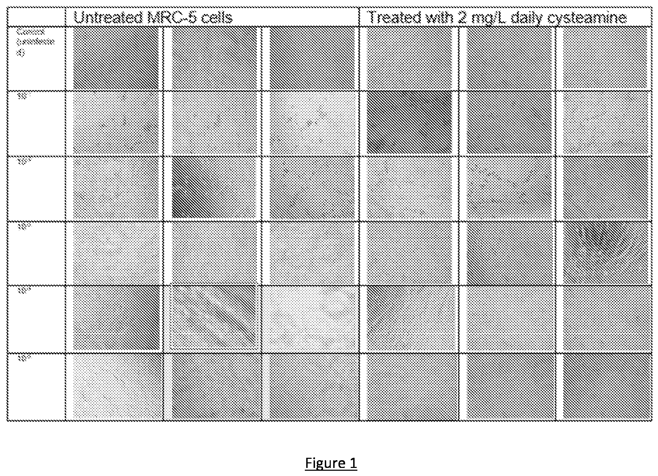

Figure 1: Representative phase contrast images (x 200 magnification) of MRC-5 lung fibroblast cell controls (uninfected) or infected with a dose-titration of hCoV 229E after 4 days infection at 33°C in Eagle's Minimum Essential Medium (EMEM) with 1% serum. Highlighted images (ie images in middle and right-hand side boxes for results of untreated cells at 10⁻⁴ and 10⁻⁵ dilutions) were of wells where there was complete destruction of the MRC-5 cell monolayer. This destruction was seen in some of the wells at 10⁻⁴ to 10⁻⁷ viral dilution- infected in untreated cells and not seen at all in any of the cysteamine treated cells.
Figure 2: Scoring sheet for a 96 well plate immunoperoxidase assay practiced on viral infected MRC-5 fibroblast cells with no cysteamine treatment.
Figure 3: Scoring sheet for a 96 well plate immunoperoxidase assay practiced on viral infected MRC-5 fibroblast cells with 1mg/L cysteamine treatment.
Figure 4: Scoring sheet for a 96 well plate immunoperoxidase assay practiced on viral infected MRC-5 fibroblast cells with 2mg/l cysteamine treatment.
Figure 5: Scoring sheet for a 96 well plate immunoperoxidase assay practiced on viral infected A549 lung epithelial cells no cysteamine treatment.
Figure 6: Scoring sheet for a 96 well plate immunoperoxidase assay practiced on viral infected A549 lung epithelial cells with 1mg/L cysteamine treatment.
Figure 7: Scoring sheet for a 96 well plate immunoperoxidase assay practiced on viral infected A549 lung epithelial cells with 2mg/l cysteamine treatment.
Figure 8: Modulation of IL-6 and IFNβ responses in A549 airway epithelial cells to infection with human coronavirus 229E by cysteamine.
Figure 9: Effect of cysteamine free base on glycine utilisation by MRC-5 fibroblasts (at 4 h)
Figure 10: Cysteamine PLP thiazolidine formation
Figure 11: An overview of the various actions of cysteamine as a treatment for viral infection. * = 14 days of cysteamine therapy adjunct to standard of care therapy (SOCT) significantly reduced WBC and CRP levels (Novabiotics CARE-CF1 phase 2b clinical trial) in acute infectious pulmonary exacerbations of cystic fibrosis versus SOCT alone.
Figure 12: Effect of Cysteamine application on the amount of hCoV 229E detected in the supernatant of infected A549 lung epithelial cells.
Figure 13: Single and multi-dose titration of cysteamine on Vero E6 MESO cells infected with SARS CoV-2 (SARS-CoV-2-CVR-Gla-1). White = cell damage/Black = intact cell monolayer. + = Positive infection control (no treatment), - = Uninfected cells. IC₅₀ of 21.36 for single dose.

### Detailed Description

### Definitions

"Virus" or "viral infection" relates to any virus in the context of the present invention. As the invention provides therapeutic utility principally via inhibition of glycine decarboxylase, and all viruses require their hosts to utilise this enzyme in order to enable viral replication, virus and viral infection can relate to any virus. Consequently, the virus or viral infection can be a member *Duplodnaviria, Monodnaviria, Riboviria, Varidnaviria,* or any combination thereof. The virus can therefore be any one or combination of the following:- a Ribovirus, an influenza, ebola, coronavirus, hepatitis C, hepatitis E , West Nile Fever, Norovirus, Rotavirus, Polio, rabies, measles, rhinovurus. Preferably the virus is a coronavirus. The virus can be SARS-CoV, MERS-CoV , SARS-CoV-2, HCoV-299E, HCoV-OC43, HCoV-NL63, CoV-HKU1, Human Coronavirus 229E (ATCC No. VR/740) or any combination thereof. The virus is most preferably SARS-CoV-2.

### Example 1: Cysteamine Treatment of Coronavirus-Infected Pulmonary Cells

The effect of treatment of coronavirus-infected pulmonary cells with cysteamine was studied. Two separate pulmonary cell lines (ie A549 and MRC-5) were used as the basis for the study. A549 (sample obtained from deposit, deposited under ATCC CCL-185) being a human lung epithelial cell line, whilst MRC-5 (sample obtained from deposit, deposited under ATCC CCL-171) being a human lung fibroblast cell line. Prior to viral challenge cells were grown at 37°C in 5% CO₂ atmosphere in growth media (EMEM + 10% FBS + pen-strep for MRC-5 cells/F-12 with Kaighn's modification + 10% FBS + pen-strep) to 70-80% confluency. Immediately prior to viral challenge, cells were washed in Hanks balanced salt solution and media was replaced with 100 µl challenge medium per well (EMEM for MRC-5 and F-12 for A549) which contained 1% serum. A separate plate of cells was used for each cysteamine treatment concentration as well as one for no treatment. Cells were incubated at 33°C in a 5% CO₂ atmosphere and treated daily with the same concentration of cysteamine until the 5th day when immunoperoxidase assay was conducted according to the protocol described in Lambert et al., 2008. 4 wells at a time were infected with a human coronavirus hCoV 229E (a coronavirus related to SARS-CoV-2, obtained from a deposit, deposited under ATCC VR/740), the concentration of virus diluted with each subsequent 4 well-group. It was determined that the stock inoculum from which each dilution was derived (determined in the untreated cells) was about 1.12 x 10¹⁴ pfu/ml.

All the infected cell cultures were treated with daily doses of cysteamine free-base. Two dosing regimens were tested on the infected cell cultures; an application of 1mg/L cysteamine once a day for 5 days was applied one set of plates, and an application of 2mg/L cysteamine once a day for 5 days was applied to another set of plates (5 microlitres of 20 x stock into 100 microlitres on the plate, with controls being provided with a mock treatment). The above procedure was carried out in quadruplicate (ie in four parallel wells, as the above was practiced in a 96 well plate).

The effect of the treatment of each infected cell culture with each dosing regimen on viral infection was assayed by microscopic examination of the cultured cells when compared to the same infected cell cultures over the same period but in the absence of cysteamine treatment. Uninfected cell lines (treated and untreated with cysteamine) were provided as controls. Viral titre of the undiluted stock of virus used as the originator stock for the study and as provided by the depository institute was determined as the 50% tissue culture infectivity dose (TCID₅₀: ie the number of viruses required to cause cytopathology in 50% of wells; a value that can be converted into the viral titre - pfu/ml by multiplying by 0.7) following immunoperoxidase assay (Lambert, et al, 2008, Methods in Molecular Biology, 454:93-102). From this undiluted stock, the range of concentrations of virus were prepared; 16 dilutions extending form a 10-1 to a 10-16 dilutions by volume from the original stock was prepared and used in the study.

### Results Summary: Results of Treatment of Coronavirus-Infected Pulmonary Cells

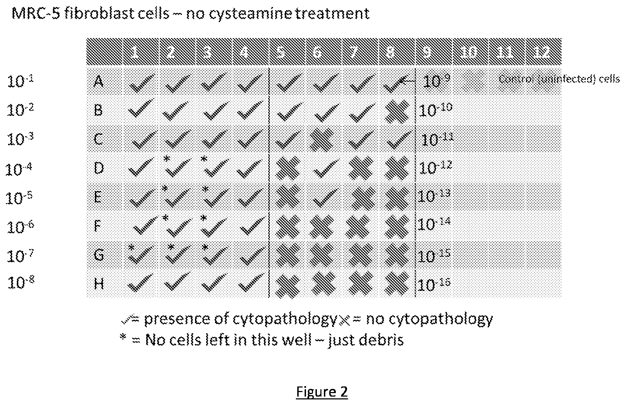

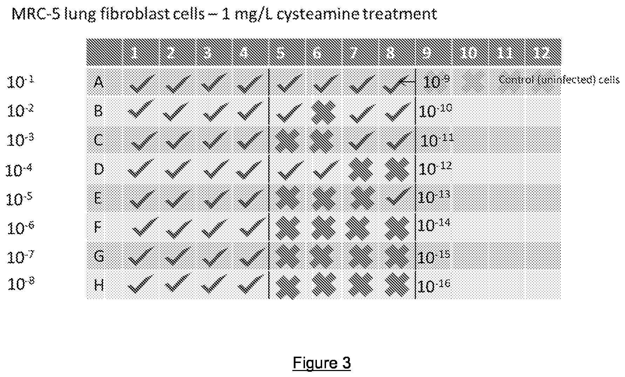

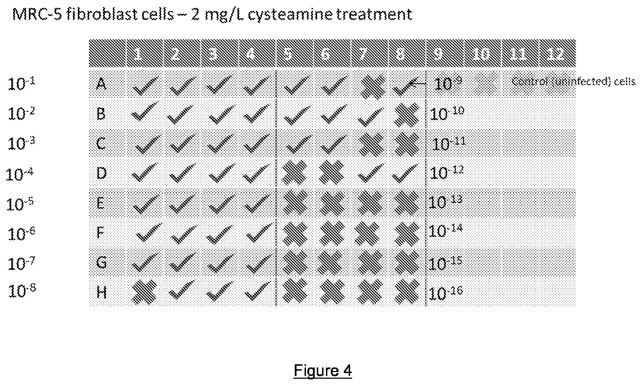

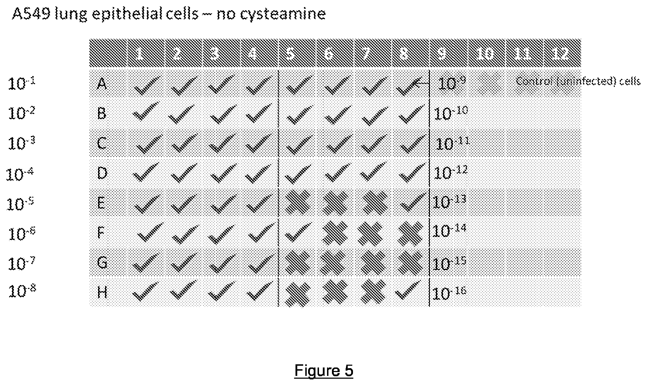

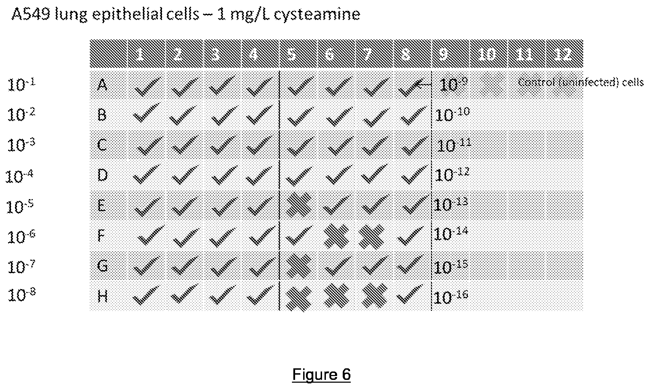

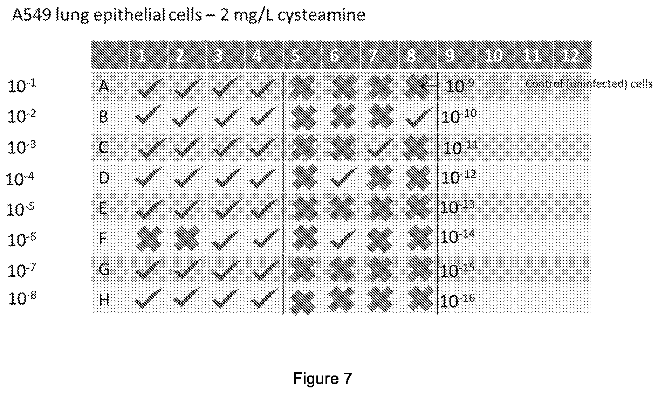

Figures 2 to 7 show the scoring sheets for the immunoperoxidase assays described above. A "tick" indicates the identification of the presence of a cytopathology. A "cross" indicates no cytopathology was identified. Those wells marked with an asterisk indicate those where no cell was identified in the wells, only cellular debris.

Application of the 2 mg/L cysteamine daily for 5 days was shown to confer protection against infection with hCoV 229E in both the A549 and MRC-5 cell lines over all concentrations of virus used in the study. Figure 1 provides the results of microscopic examination on day 4 of this study when applied to MRC-5 cells. It can be seen that in two of the shown three repeats of the test, untreated cell lines at 10⁻⁴ and 10⁻⁵ dilutions of the virus showed a complete destruction of the pulmonary cell monolayer (although not shown, the same was found for dilutions 10⁻⁶ and 10⁻⁷). This can be contrasted with the visible intact monolayer exhibited in all of the MRC-5 cell lines treated with 2mg/l of the cysteamine throughout all concentrations of the virus of the study (although only concentrations10⁻¹ to 10⁻⁵ are shown in Figure 1). The same results shown in figure 1 were found for A549 cell lines tested.

The high level of pfu/ml of the inoculum demonstrates that the concentrations tested contained a very high number of infectious 229E virions representing a physiologically very heavy viral load of infection.

| | pfu/ml | |
|---|---|---|
| | **A549 Cells** | **MRC-5 Cells** |
| 0 mg/L cysteamine | 2.21 x 10¹⁴/ml | 1 x 10^{12.5}/ml |
| 1 mg/L cysteamine | 3.94 x 10¹⁵/ml | 1 x 10^{12.5}/ml |
| 2 mg/L cysteamine | 7 x 10⁹/ml | 1 x 10¹¹⁻⁷¹/ml |

**Table 1:** values for pfu/ml established in those wells receiving a cysteamine treatment and infected with hCoV 229E.

A lower pfu/ml value, relative to that of the control in which cysteamine was not applied, demonstrates that cysteamine can reduce viral load in both cell types at 2 mg/L (which is physiologically achievable *in vivo*)*.*

These results clearly demonstrate an ability for cysteamine to both reduce viral load in a coronavirus infection and to provide cell-protective role against viral induced cell death.

### Example 2: The reduction in viral load is via cysteamine's action on the glycine cleavage pathway

To investigate how cysteamine is reducing viral load, the following experiment looked at cytokines which are modulated by the glycine cleavage system.

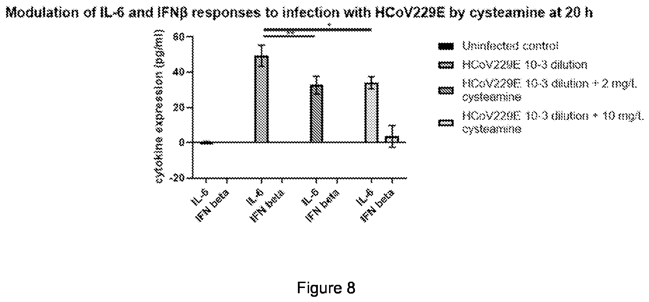

Figure 8 shows the interleukin-6 (IL-6) and interferon beta (IFNβ) cytokine responses produced by A549 airway epithelial cells in response to infection with human coronavirus 229E with and without single treatments of cysteamine at the concentrations shown after incubation for 20 h at 37°C. Cell monolayers were treated in triplicate with or without 10⁻³ dilution of stock HCoV 229E virus as stated and with or without single doses of cysteamine as shown at therapeutically achievable concentrations of 2 or 10 mg/L. After 20 hours the cell supernatants were harvested and cytokine concentrations were measured using either the R and D Systems IFN Beta Quantikine kit or the IL-6 Duoset kit immunoassays. Results confirm that HCoV 229E, in common with all coronaviruses, elicits little IFNβ alone as none is detected in response to viral challenge. However, in the presence of 10 mg/L cysteamine IFNβ is detected, demonstrating an increase in type I interferon signalling mediated by cysteamine. Viral challenge does elicit IL-6 secretion, but treatment with single doses of cysteamine significantly reduced IL-6 secretion compared with viral infection alone as determined by one-way ANOVA and Tukey's post hoc analysis. Interferon beta signalling can reduce IL-6 pro-inflammatory responses.

**Results summary:** These results show that cysteamine treatment modulates cytokines downstream from the glycine cleavage pathway. The glycine cleavage pathway feeds into the biosynthetic pathways for purines and pyrimidines, the building blocks for nucleotide synthesis. When a cell is infected by a virus, purine and pyrimidine levels are reduced as these are being used for viral replication. These modulated cytokines are induced in response to this reduction in pyrimidines/purines, as a host response. The inventors hypothesise that cysteamine is acting on the enzyme glycine decarboxylase in the glycine cleavage pathway to cause this cytokine modulation. By acting on glycine decarboxylase, the actions of cysteamine are two-fold: 1) it reduces viral load by inhibiting the production of pyrimidines and purines which could otherwise be used for viral replication; and 2) as a result of this reduction in these nucleotide building blocks, boosts the cell's natural response to viral attack by inducing cytokines which help viral clearance. This multi-pronged action of cysteamine is summarised in Figure 11. Cysteamine potently inhibits the enzyme glycine decarboxylase in host mitochondria. The inhibition of *de novo* purine and pyrimidine synthesis reduces the number of building blocks required for the replication of viruses during infection. It also enhances the Interferon beta (IFN-β) response to viral infection, thereby encouraging the resolution phase of antiviral immune response. At the same time cysteamine can reduce the white blood cell count (WBC) and C-reactive protein (CRP) during infection reducing inflammation.

### Example 3: Effect of cysteamine on glycine utilisation by MRC-5 fibroblasts Cell challenge

MRC-5 airway fibroblast cells were grown to 90% confluence in 2 x 12-well plates in EMEM + 10% FBS. Cell culture media was replaced with:
Plate 1

| | |
|---|---|
| 1. | 3 x 1 ml media alone (EMEM + 10% FBS) |
| 2. | 3 x 1 ml media + insulin (10 mg/L) |
| 3. | 3 x 1 ml media + added glycine (300 µM) |
| 4. | 3 x 1 ml media + glycine + insulin |

Plate 2

As above, but with the addition of cysteamine to a final concentration of 10 mg/L

Cells were then incubated at 37°C and 5% CO₂ for 4 h. After incubation cell supernatants were harvested and transferred to labelled cryotubes and stored at -80°C prior to assessment of glycine content. Insulin strongly stimulates glycine utilisation via glycine decarboxylase.

### Glycine assessment

The supernatants were thawed prior to determining the glycine concentration using the fluorometric glycine assay kit from Abcam (Ab211100).

Manufacturer's instructions were followed and fluorometric assessment of glycine concentration in supernatants and standards was performed on the Biotek Synergy plate reader using 96-well black-walled plates at 535/587 nm (ex/em).

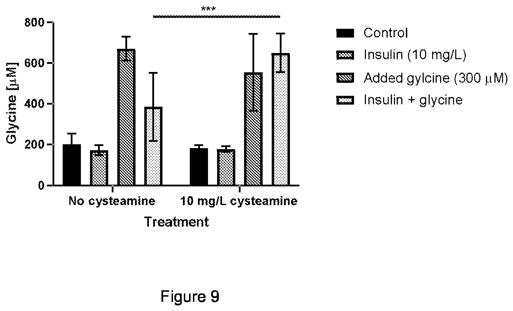

**Results summary:** The results are shown in Figure 9. These provide further confirmation that cysteamine potently inhibits the enzyme glycine decarboxylase in host cells. Here insulin drives the decarboxylation of exogenous glycine in the media of MRC-5 fibroblasts. The utilisation of which is blocked by the addition of 10 mg/L cysteamine

### Example 4: Cysteamine PLP thiazolidine formation: Reaction of cysteamine to Pyridoxal-5' phosphate (PLP) cofactor

Glycine Decarboxylase (GLDC) activity depends upon PLP cofactor. Pyridoxal-5-phosphate (PLP) is yellow green in aqueous solution with an absorption maxima at 388nm. A reaction with cysteamine will form a thiazolidine compound with a blue-shifted absorbance maxima at 320nm.

10 ml equimolar solutions of 20 mM pyridoxal 5'-phosphate monohydrate (Sigma: 82870), 20mM cysteamine free base (CFB), 20mM cysteamine bitartrate (CBT) and 20mM cystamine dihydrochloride (CTM) were prepared in carbonate-bicarbonate buffer pH 7.4

In a 96-well microtitre plate 6 x replicates of 100 µl total volume containing each of the following reaction mixes were prepared:

| | |
|---|---|
| 1. | Buffer only |
| 2. | PLP only (2 mM final concentration) |
| 3. | CFB only (2 mM) |
| 4. | CBT only (2 mM) |
| 5. | CTM only (2 mM) |
| 6. | CFB (2 mM) + PLP (2 mM) |
| 7. | CBT (2 mM) + PLP (2 mM) |
| 8. | CTM (2 mM) + PLP (2 mM) |

Plates were mixed gently by tapping and incubated for 1 h in the dark at room temperature prior to full spectral scan (300-700nm) performed on Biotek Powerwave and the absorbances recorded. Absorbances at 390 and 320 nm compared for reaction components and products.

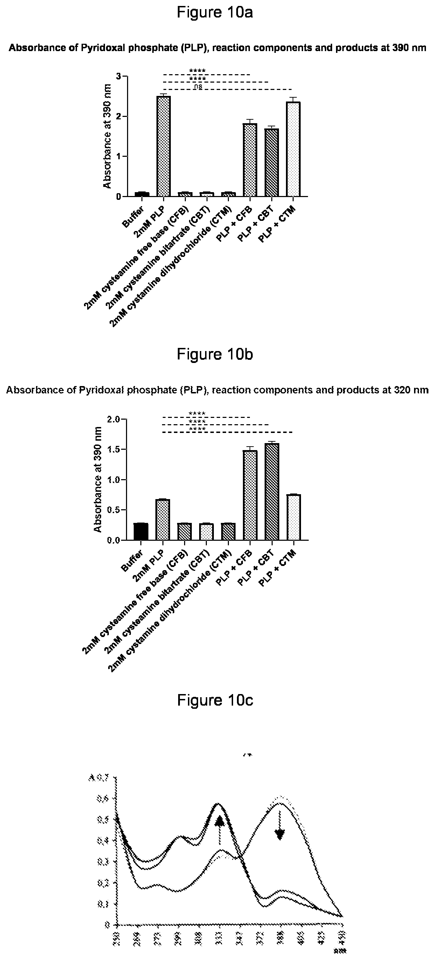

Figure 10 demonstrates a reaction between cysteamine and PLP. Panel A and B confirm the reaction in physiological buffer with equimolar concentrations of PLP, cysteamine free base, cysteamine bitartrate and the disulphide, cystamine dihydrochloride. There is a significant reduction (**** p=<0.0001) in the absorbance at 390 nm caused by cysteamine free base and bitartrate (Brown-Forsythe and Welch ANOVA), non-significant reduction caused by the addition of cystamine (n=6). At 320 nm there is a reduction in the absorbance in PLP alone than at 390 nm and there is a significant difference between PLP alone and the reaction products caused by the addition of cysteamine free base, cysteamine bitartrate and cystamine dihydrochloride.

**Results summary:** Cysteamine is shown to react with the GLDC co-factor PLP. The inventors hypothesise cysteamine may inhibit GLDC via access to, and reaction with the PLP co-factor rather than directly with the GLDC enzyme. Cysteamine forms a thiazolidine ring with PLP and as shown in Figure 10 this causes a blue shift in absorbance maxima from 390 nm to 320 nm (as shown in panel 10c). Both cysteamine free base and cysteamine bitartrate, and to a lesser extent, cystamine dihydrochloride, react with PLP.

### Example 5: Cysteamine lowers virus titer

Following on from Example 1, to further investigate the efficacy of cysteamine to reduce viral titer, cysteamine was applied to infected A549 lung epithelial cells and viral load was measured by PCR.

The effect of treatment of coronavirus-infected pulmonary cells with cysteamine was studied using A549 (sample obtained from deposit, deposited under ATCC CCL-185) being a human lung epithelial cell line. Prior to viral challenge A549 cells were grown at 37°C in 5% CO₂ atmosphere in growth media F-12 with Kaighn's modification + 10% FBS + pen-strep [F-12K]) to 70-80% confluency in F12-K in tissue-culture-treated 96-well plates. Immediately prior to viral challenge, cells were washed in Hanks balanced salt solution and media was replaced with 100 µl challenge medium per well (F-12K) which contained 1% serum. A separate plate of cells was used for each cysteamine treatment concentration as well as one for no treatment. Cells were incubated at 33°C in a 5% CO₂ atmosphere and treated daily with the same concentration of cysteamine (0, 1 or 2 mg/L) until the 5th day when supernatants were removed and viral RNA was extracted using the Qiagen viral RNA mini kit and RNA was frozen at -85°C until required. Viral RNA was reverse transcribed using Superscript IV reverse transcriptase (Thermo-Fisher)).

Quantitative (real-time) polymerase chain reaction (qPCR) was carried out using PrecisionPLUS qPCR Master Mix (PrimerDesign) and forward and reverse oligonucleotides (229E Sense & 229E Antisense) specific to the hCoV 229E M protein gene. Amplification was carried out using a MiniOpticon Real-Time PCR system (Bio-Rad) and data was analysed using Opticon Monitor software (Bio-Rad). Analysed samples had been exposed to 10⁻³ (1.12 x 10¹² pfu/ml), 10⁻⁴ (1.12 x 10¹¹ pfu/ml) and 10⁻⁵ (1.12 x 10¹⁰ pfu/ml) of hCoV 229E and conducted from quadruplicate technical replicates. The number of copies of the 229E M protein gene were determined from a standard curve containing known number of copies of the 229E M protein gene.

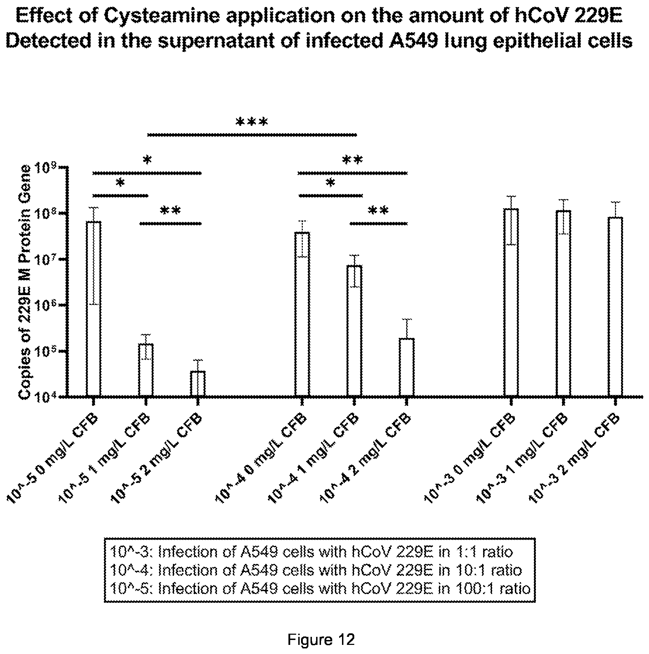

**Results Summary:** The results are shown in Figure 12. These results demonstrate that cysteamine at low concentrations reduces viral load as measured by number of copies of the 229E protein.

### Example 6: The activity of cysteamine on SARS CoV-2

### Materials:

a) Assays were performed in Vero E6 MESO cell line, which is a subclone of Vero E6 cell line based on susceptibility to SARS-CoV-2
b) SARS-CoV-2-CVR-Gla-1 strain used in this study was originally Isolated from a patient sputum sample, and it contains D614G mutation in Spike gene (GISAID accession:
   EPI_ISL_461705).
c) Cysteamine was dissolved in water, aliquoted, and stored at -80C.

### Method:

Vero E6 MESO cells were seeded in 96-well plates and cultured overnight. The following day, 2-fold serial dilutions of cysteamine starting at 256 mg/L were prepared in (after addition of the virus the final compound concentrations are 128 mg/L, 64 mg/L, 32 mg/L, 16 mg/L, 8 mg/L, 4 mg/L, 2 mg/L, 1 mg/L, 0.5 mg/L, 0.25 mg/L, 0 µM). Serial dilutions of the compounds were added to cells as depicted in the plate layout below, and infected with one dose of SARS-CoV-2.

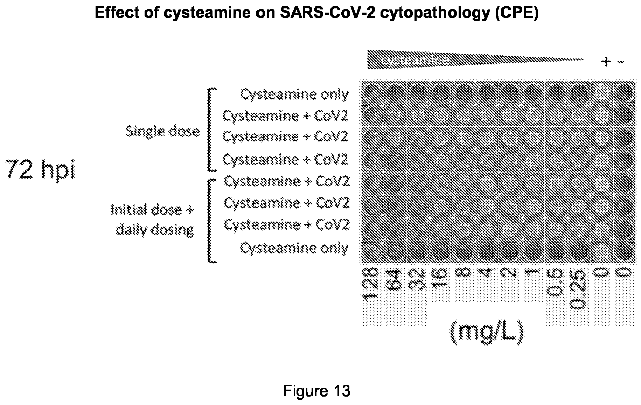

Plates were incubated at 37°C. Every 24 h one plate was fixed and stained. Prior to fixing supernatant from the cells was harvested into Trizol. A daily dose of cysteamine of 2 mg/L final was added to wells 1 to 11 in rows E to H in the remaining plates. The remaining half of the plate (i.e. rows A to D) was left untouched. Daily dosing regimen for each plate and timepoint is presented in Figure 13. Plates were scanned in a plate reader to quantitate the levels of CPE.

**Results Summary: The results are shown in** **Figure 13****.** These results demonstrate that cysteamine reduced the CPE (Cytopathology) induced by SARS-CoV-2. At 72 hours post-infection it is evident that cysteamine can protect Vero cells from infection. At therapeutically-achievable doses this effect is modest, but rises to almost complete protection of the cell monolayer at 128 mg/L, which persists at later time points. Cysteamine had an IC₅₀ of 21.36 at 72 hours post infection.

## Claims

1. Cysteamine or cystamine, or a pharmaceutically salt thereof, for use in a method of treatment of viral infection in a subject, wherein the method comprises inhibiting the activity of glycine decarboxylase.

2. Cysteamine or cystamine for use as claimed in claim 1, for use in simultaneously treating a viral and a bacterial infection.

3. A composition comprising cysteamine or cystamine or a pharmaceutically acceptable salt thereof, and an additional inhibitor of glycine cleavage for use in a method of treating a viral infection in a subject, wherein the method comprises cysteamine or cystamine inhibiting the activity of glycine decarboxylase.

4. The composition for use of claim 3, wherein the inhibitor is bicarbonate.

5. The composition for use of claims 3-4, wherein the composition is a pharmaceutical composition additionally comprising one or more pharmaceutically acceptable excipient.

6. Cysteamine or cystamine for use as claimed in any claims 1 to 2, or a composition for use in any of claims 3 to 5 further comprising an additional antiviral agent.

7. Cysteamine or cystamine for use in accordance with claims 1-2 or the composition for use in accordance with claims 3-6, wherein the viral infection is a coronavirus infection optionally wherein the coronavirus is SARS-CoV-2.

8. Cysteamine or cystamine for use in accordance with claims 1-2, 6 or 7 or the composition for use in accordance with claim 3-7, wherein the treatment is prophylactic treatment.

9. Cysteamine or cystamine for use in accordance with claims 1-2, 6, 7 or 8 or the composition for use in accordance with claims 3-8, wherein the subject has a greater than normal glycine decarboxylase activity.

10. Cysteamine or cystamine for use in accordance with claims 1-2 or 6-9 or the composition for use in accordance with claims 3-9, wherein the subject has a plasma concentration of glycine that is lower than or equal to 300 µmol/L.

11. Cysteamine or cystamine for use in accordance with claims 1-2,or 6-10; or the composition for use in accordance with claims 3-10, wherein the subject is a diabetic, obese, suffers from dementia, is elderly, or a combination thereof.

12. The composition for use in accordance with claims 3-11 wherein the cysteamine or cystamine is administered separately, sequentially or simultaneously with the inhibitor of glycine cleavage.

13. Cysteamine or cystamine for use in accordance with claims 1-2 or 6-12 or the composition for use in accordance with claims 3-12 wherein the composition is administered to the lungs.

14. Cysteamine for use in a method of treating hyperinflammation in a subject with pneumonia caused by viral or bacterial infection.

15. Cysteamine for use in accordance with claim 14, wherein the method is prophylactic.

## Patentansprüche

1. Cysteamin oder Cystamin oder pharmazeutisches Salz davon zur Verwendung in einem Verfahren zur Behandlung einer Virusinfektion bei einem Subjekt, wobei das Verfahren Hemmen der Aktivität von Glycin-Decarboxylase umfasst.

2. Cysteamin oder Cystamin zur Verwendung nach Anspruch 1, zur Verwendung bei der gleichzeitigen Behandlung einer viralen und einer bakteriellen Infektion.

3. Zusammensetzung, umfassend Cysteamin oder Cystamin oder ein pharmazeutisch unbedenkliches Salz davon und einen zusätzlichen Hemmstoff der Glycinspaltung zur Verwendung in einem Verfahren zum Behandeln einer Virusinfektion bei einem Subjekt, wobei das Verfahren Cysteamin oder Cystamin umfasst, das die Aktivität von Glycin-Decarboxylase hemmt.

4. Zusammensetzung zur Verwendung nach Anspruch 3, wobei der Hemmstoff Bicarbonat ist.

5. Zusammensetzung zur Verwendung nach Anspruch 3-4, wobei die Zusammensetzung eine pharmazeutische Zusammensetzung ist, die zusätzlich einen oder mehrere pharmazeutisch unbedenkliche Hilfsstoffe umfasst.

6. Cysteamin oder Cystamin zur Verwendung nach einem der Ansprüche 1 bis 2 oder Zusammensetzung zur Verwendung nach einem der Ansprüche 3 bis 5, ferner umfassend ein zusätzliches antivirales Mittel.

7. Cysteamin oder Cystamin zur Verwendung nach Anspruch 1-2 oder Zusammensetzung zur Verwendung nach Anspruch 3-6, wobei es sich bei der Virusinfektion um eine Coronavirusinfektion handelt, wobei es sich bei dem Coronavirus gegebenenfalls um SARS-CoV-2 handelt.

8. Cysteamin oder Cystamin zur Verwendung nach Anspruch 1-2, 6 oder 7 oder Zusammensetzung zur Verwendung nach Anspruch 3-7, wobei die Behandlung eine prophylaktische Behandlung ist.

9. Cysteamin oder Cystamin zur Verwendung nach Anspruch 1-2, 6, 7 oder 8 oder Zusammensetzung zur Verwendung nach Anspruch 3-8, wobei das Subjekt eine überdurchschnittliche Glycin-Decarboxylase-Aktivität aufweist.

10. Cysteamin oder Cystamin zur Verwendung nach Anspruch 1-2 oder 6-9 oder Zusammensetzung zur Verwendung nach Anspruch 3-9, wobei das Subjekt eine Plasmakonzentration von Glycin aufweist, die kleiner oder gleich 300 pmol/l ist.

11. Cysteamin oder Cystamin zur Verwendung nach Anspruch 1-2 oder 6-10; oder Zusammensetzung zur Verwendung nach Anspruch 3-10, wobei das Subjekt ein Diabetiker ist, fettleibig ist, an Demenz leidet, es sich um einen älteren Menschen handelt oder eine Kombination davon.

12. Zusammensetzung zur Verwendung nach Anspruch 3-11, wobei das Cysteamin oder Cystamin getrennt, nacheinander oder gleichzeitig mit dem Hemmstoff der Glycinspaltung verabreicht wird.

13. Cysteamin oder Cystamin zur Verwendung nach Anspruch 1-2 oder 6-12 oder Zusammensetzung zur Verwendung nach Anspruch 3-12, wobei die Zusammensetzung in die Lunge verabreicht wird.

14. Cysteamin zur Verwendung in einem Verfahren zur Behandlung von Hyperinflammation bei einem Subjekt mit Lungenentzündung, die durch virale oder bakterielle Infektion verursacht wird.

15. Cysteamin zur Verwendung nach Anspruch 14, wobei das Verfahren prophylaktisch ist.

## Revendications

1. Cystéamine ou cystamine, ou sel pharmaceutique de celles-ci, destiné(e) à être utilisé(e) dans un procédé de traitement d'une infection virale chez un sujet, ledit procédé comprenant l'inhibition de l'activité de la glycine décarboxylase.

2. Cystéamine ou cystamine destinée à être utilisée selon la revendication 1, destinée à être utilisée dans le traitement simultané d'une infection virale et d'une infection bactérienne.

3. Composition comprenant la cystéamine ou la cystamine ou un sel acceptable pharmaceutiquement de celles-ci, et un inhibiteur supplémentaire du clivage de la glycine destinée à être utilisée dans un procédé de traitement d'une infection virale chez un sujet, ledit procédé comprenant l'inhibition par la cystéamine ou la cystamine de l'activité de la glycine décarboxylase.

4. Composition destinée à être utilisée selon la revendication 3, dans laquelle l'inhibiteur est le bicarbonate.

5. Composition destinée à être utilisée selon les revendications 3 à 4, ladite composition étant une composition pharmaceutique comprenant en outre un ou plusieurs excipients acceptables pharmaceutiquement.

6. Cystéamine ou cystamine destinée à être utilisée selon l'une quelconque des revendications 1 à 2, ou une composition destinée à être utilisée selon l'une quelconque des revendications 3 à 5 comprenant en outre un agent antiviral supplémentaire.

7. Cystéamine ou cystamine destinée à être utilisée selon les revendications 1 à 2 ou composition destinée à être utilisée selon les revendications 3 à 6, ladite infection virale étant une infection par un coronavirus, éventuellement ledit coronavirus est le SARS-CoV-2.

8. Cystéamine ou cystamine destinée à être utilisée selon les revendications 1 à 2, 6 ou 7 ou composition destinée à être utilisée selon les revendications 3 à 7, ledit traitement étant un traitement prophylactique.

9. Cystéamine ou cystamine destinée à être utilisée selon les revendications 1 à 2, 6, 7 ou 8 ou composition destinée à être utilisée selon les revendications 3 à 8, ledit sujet présentant une activité de la glycine décarboxylase supérieure à la normale.

10. Cystéamine ou cystamine destinée à être utilisée selon les revendications 1 à 2 ou 6 à 9 ou composition destinée à être utilisée selon les revendications 3 à 9, le sujet présentant une concentration plasmatique de la glycine inférieure ou égale à 300 µmol/l.

11. Cystéamine ou cystamine destinée à être utilisée selon les revendications 1 à 2 ou 6 à 10 ; ou composition destinée à être utilisée selon les revendications 3 à 10, ledit sujet étant diabétique, obèse, souffrant de démence, âgé ou une combinaison de ceux-ci.

12. Composition destinée à être utilisée selon les revendications 3 à 11, dans laquelle la cystéamine ou la cystamine est administrée séparément, séquentiellement ou simultanément avec l'inhibiteur du clivage de la glycine.

13. Cystéamine ou cystamine destinée à être utilisée selon les revendications 1 à 2 ou 6 à 12 ou composition destinée à être utilisée selon les revendications 3 à 12, ladite composition étant administrée aux poumons.

14. Cystéamine destinée à être utilisée dans un procédé de traitement de l'hyperinflammation chez un sujet atteint de pneumonie causée par une infection virale ou bactérienne.

15. Cystéamine destinée à être utilisée selon la revendication 14, ledit procédé étant prophylactique.
